# EUROPEAN PATENT SPECIFICATION

(11) **EP 1 778 183 B1**
(45) Date of publication and mention of the grant of the patent: **27.06.2018**
(21) Application number: 05784837.6
(22) Date of filing: 17.08.2005
(51) Int. Cl.: A61K 9/08, A61K 31/4525, A61K 31/452, A61K 31/724

(54) **LIQUID PAROXETINE COMPOSITIONS**
FLÜSSIGE PAROXETINZUSAMMENSETZUNGEN
COMPOSITIONS LIQUIDES DE PAROXETINE

(30) Priority: 18.08.2004 US 602338 P
(43) Date of publication of application: 02.05.2007
(73) Proprietor: Synthon B.V., 6545 CM Nijmegen (NL)
(72) Inventor: KALMOUA, Faysal, NL-5348 AD Oss (NL); HOLMDAHL, Lisa, Karin, NL-6821 EG Arnhem (NL); VAN DALEN, Frans, NL-6524 CK Nijmegen (NL)
(74) Representative: Mendivil Gil, Maria Dolores
(86) International application number: PCT/EP2005/009035
(87) International publication number: WO 2006/018318

(56) References cited:
- EP-A- 1 304 109
- WO-A-02/085360
- US-A1- 2003 032 809

## Description

### BACKGROUND OF THE INVENTION

### 1. Field of the Invention

The present invention relates to liquid paroxetine compositions, especially pharmaceutical formulations and dosage forms, and to processes of manufacturing and using the same.

### 2. Description of the Related Arts

US Patent 4,007,196 describes certain compounds that possess anti-depressant activity. One specific compound mentioned in this patent is known as paroxetine and is represented by the following formula.

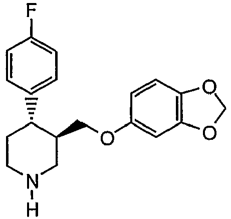

Paroxetine hydrochloride has been approved for treating, *inter alia,* depression in humans and is being marketed around the world under such brand names as Paxil® and Seroxat^{tm} by SmithKline Beecham, now Glaxo SmithKline. Marketed dosage forms thus far include immediate release tablets, extended release tablets, capsules and suspensions. The drug substance is typically paroxetine hydrochloride hemihydrate as disclosed in US Patent 4,721,723 and EP 223403, although paroxetine hydrochloride anhydrate is also now marketed as well.

US Patent 5,874,447 describes paroxetine sulfonate salts, including paroxetine methane sulfonate also known as paroxetine mesylate. These sulfonate salts have advantageous properties in comparison to the prior known salts, including the hydrochloride salts. For example, the sulfonate salts have high water solubility and good thermal stability, making them useful in forming a commercial paroxetine dosage form. The paroxetine methane sulfonate (or mesylate) has also been approved for sale in many countries and is marketed as an immediate release tablet; e.g. in the U.S. as Pexeva® by Synthon Pharmaceuticals, Inc.

Tabletted paroxetine compositions are of limited use in patients which have mental or physiological problems with swallowing tablets. For such patients, a drinkable suspension of paroxetine hydrochloride (10 mg/5 ml) is on the market. Inactive ingredients consist of polacrilin potassium, microcrystalline cellulose, propylene glycol, glycerin, sorbitol, methyl paraben, propyl paraben, sodium citrate dihydrate, citric acid anhydrate, sodium saccharin, flavorings, FD&C yellow no. 6 and simethicone emulsion. The suspension must be well shaken before the use; failing to do so can result in an improper dose being administered.

EP 1304109 (US 2003/078285) suggests an oral liquid pharmaceutical composition comprising paroxetine or its salt, water, and a basic compound to rise a pH to >7, preferably 8-10, which helps to overcome drug bitterness. The basic compound is selected from sodium chloride or a sodium salt of a weak acid, such as sodium bicarbonate, sodium carbonate, sodium citrate, disodium phosphate, or trisodium phosphate. However, such a composition is only practical for low concentrations of paroxetine due to the fact that paroxetine and its salts with weak acid anions are generally only slightly soluble in aqueous solutions having a pH > 7. Thus, higher concentrations will tend to be unstable with precipitation of paroxetine. EP1304109, US2003032809, WO02085360 disclose varied liquid paroxetine compositions. It would be desirable to have a liquid paroxetine dosage form that can be taken directly or after being diluted to a preferred concentration or volume. In particular, it would be desirable to have a homogeneous liquid for which care in shaking before use is not needed.

Several problems exist, however, in making such a liquid that would have to be solved simultaneously. For example:
1) the inherent bitter taste of paroxetine should be lessened or avoided in order to make the liquid more palatable as an oral dosage form;
2) sufficient stability of the liquid, both physical and chemical, during long-term storage would be desirable; and
3) the liquid should be compatible with a variety of diluent medium.

### SUMMARY OF THE INVENTION

Now, it has surprisingly been discovered that paroxetine may be formulated into a liquid having a concentration of 5 mg or more of paroxetine per ml that is sufficiently stable; compatible with taste masking agents such as sweeteners, chelating agents, cyclodextrines, complex forming agents and/or taste blocking agents as well as with other excipients; and compatible with common dilution media, by the use of a select group of solvents.

Accordingly, the present invention relates to a liquid paroxetine composition, comprising (a) paroxetine mesylate in a concentration of at least 5 mg/ml expressed in terms of paroxetine base; (b) a non-carbohydrate sweetener; and (c) a solvent which comprises 60-100% of an organic solvent component comprising ethanol, polyol, or mixtures of one or more thereof, and 0-40% of water. The concentration of the paroxetine compound is typically in the range of 10-50 mg/ml, calculated as paroxetine base. The non-carbohydrate sweetener is typically present in an amount of 0.1 to 20 mg/ml. The solvent typically contains ethanol and a polyol and optionally water. The composition can be administered "as is" or it can be diluted before administration, e.g. for taste and/or convenient volume, etc.

### DETAILED DESCRIPTION OF THE INVENTION

Soluble paroxetine salts tend to have an inherently bitter taste. Thus, a taste-masking agent is necessary in making pharmaceutically useful oral liquid compositions. Using a sweetener, optionally in a combination with a flavorant, is one possibility for masking the bitter taste. However it was discovered that common carbohydrate sweeteners (mannitol, sorbitol) are not "sweet enough," i.e. cannot sufficiently mask the bitter taste of paroxetine salts. Typically amounts higher than 200 mg/ml would be necessary. A non-carbohydrate sweetener, on the other hand, can provide sufficient taste masking, but provides a new problem. In the presence of paroxetine salts, the aqueous solubility of otherwise well soluble non-carbohydrate sweeteners such as saccharin sodium decreases dramatically. Also the stability of those compositions was found to be insufficient. Paroxetine mesylate, is generally mutually incompatible with non-carbohydrate sweeteners in an aqueous solution. Surprisingly, it was discovered that using a solvent that contained at least 60% of an ethanol and/or polyol, the remainder if any being water, could overcome the problems of compatibility and stability and allows for even highly concentrated paroxetine liquid compositions to be formed.

Specifically, the "solvent" of the present invention can be a single solvent or a mixture of solvents; i.e. a solvent system. The solvent contains 60-100% of an organic component and 0-40% of water as a co-solvent. The organic component comprises a water-miscible pharmaceutically acceptable organic solvents selected from ethanol, polyol, and combinations thereof. The polyol is preferably glycerol, polyethylene glycol or propylene glycol. While ethanol can be used as the single solvent or as the sole organic solvent component, it can present evaporation concerns. Usually the ethanol, if any, comprises 75% or less, usually 50% or less, and generally 25% or less of the organic solvent component. Avoiding ethanol altogether, i.e. using only polyol(s) as the organic solvent component, is thus contemplated as an embodiment of the invention. On the other hand, ethanol provides an antimicrobial effect and thus providing some amount of ethanol can be advantageous. Accordingly, the organic solvent component generally is a mixture of ethanol with one or more polyols. Typically the weight ratio of ethanol to polyol(s) is within the range of 1:99 to 75:25, more typically 10:90 to 50:50, and in some embodiments about 15:85 wherein "about" means +/- 5; e.g., 20:80 to 10:90 of ethanol to polyol(s).

The paroxetine used in the composition of the present invention is generally the dissolved form of a pharmaceutically acceptable salt. Preferred paroxetine salts include paroxetine hydrochloride, including the various anhydrous and hydrated forms thereof, paroxetine acetate, and the paroxetine sulfonates such as paroxetine mesylate, paroxetine besylate, etc. The total concentration of paroxetine is typically within the range of 5-100 mg/ml, more typically 10-50 mg/ml, calculated as paroxetine base.

The non-carbohydrate sweetener can be any sweetening agent that is not sugar or derived from a sugar, i.e. not a carbohydrate of the general formula of CₙH₂ₙOₙ or derived therefrom. Accordingly sugar alcohols such as mannitol or sorbitol are not non-carbohydrate sweeteners. Examples of suitable non-carbohydrate sweeteners include sodium saccharin, acesulfame potassium, aspartame, sodium cyclamate, thaumatin, sucralose and mixtures thereof. The non-carbohydrate sweetener is typically used in a concentration of 0.1 to 20 mg/ml, preferably 1-10 mg/ml. A carbohydrate sweetener can also be used in addition to the non-carbohydrate sweetener if desired.

The liquid composition can additionally comprise auxiliary agents such as flavorants, e.g., mints; solubility enhancers and/or surfactants, e.g., polysorbates, cyclodextrins; pH adjustors; and/or colorants. Such compounds are generally compatible and stable in the liquid composition of the invention.

The liquid composition of the present invention generally has a pH of 7.0 or less, more preferably a pH of 6.0 or less, including about 5.5 or less. The pH is usually not lower than 3.5. Typically no pH-adjusting excipients are present except the paroxetine salt itself and the sweetener. If however, the final pH caused by the nature of the components would be above 7.0, then an acidic compound or buffer may be added to adjust the final pH of the composition below 7.0, preferably below 5.5. A suitable acidic compound is a pharmaceutically acceptable inorganic or organic acid such as hydrochloric acid or citric acid. The pH-adjusting agent, if needed, is normally added as the final ingredient to bring the composition to a final pH between 3.5 and 7.0.

In some embodiments, the composition is preferably free from antioxidants and free from preservatives.

Because the liquid composition of the present invention contains dissolved paroxetine, the drug substance is uniformly present and capable of accurate dosing without the need for perfect agitation/mixing as with the prior suspension product. Liquid composition of the present invention is generally almost always a complete solution, namely all excipients as well as the paroxetine are dissolved in the solvent. But in rare cases pharmaceutically inert and/or flavoring solids could be present; e.g. the pulp of a fruit juice may be present, etc. Typically and especially for the concentrated liquid (i.e. at least 5 mg paroxetine/ml) all ingredients are in solution.

The compositions of the present invention can be made by any conventional process of mixing the active substance and excipients and dissolving them into the proper liquid medium. The components may be dissolved in the final medium at once, or the paroxetine salt may be dissolved separately and then mixed with a solution of excipients, or solid excipients may be added into the solution of the paroxetine salt. Any possible combination of dissolving and combining of the components falls within the present invention. While it is preferred that all excipients are dissolved in the solvent, it is not strictly required, and the undissolved excipient can be removed such as by filtering to make the final liquid composition.

The liquid composition can be packaged in a variety of ways for commercial sale. For example, the paroxetine liquid composition can be packaged in a container that is equipped with or accompanied by a device for measuring or dosing the proper volume of the liquid composition. Alternatively, the paroxetine liquid composition can be packaged in unit vials or flasks, each containing a single intended paroxetine dose.

The composition of the present invention can be used to treat or prevent the following disorders: depression, obsessive compulsive disorder, major depressive disorder, alcoholism, anxiety, panic disorder, social anxiety disorder, generalized anxiety disorder, post-traumatic stress disorder, chronic pain, obesity, senile dementia, migraine, bulimia, anorexia, social phobia, pre-menstrual syndrome, adolescent depression, trichotillomania, dysthymia, substance abuse etc., hereinafter the "paroxetine-treatable disorders." Most suitably, the composition of the invention is applied for treatment of depression, obsessive compulsive disorder and/or panic disorder.

The treatment or prevention of any one or more of the paroxetine-treatable disorders is performed by administering orally the liquid paroxetine composition to an animal in need thereof, especially a mammal and in particular a human, in an effective amount. The effective amount, which includes a prophylactic amount, is generally from 0.05 to 6 mg/kg, more preferably 0.14 to 0.86 mg/kg, per day. The dose is normally taken from 1 to 6 times daily, but more usually once or twice daily, with the total amount of paroxetine administered being generally between 5 to 400 mg. Typically then, a single dose of the paroxetine liquid, either as measured/metered or as supplied in a single dose vial, etc., contains from 5 to 100 mg, usually 10 to 50 mg of paroxetine, expressed as free base. Conventional doses are 5, 10, 20, 30, and 40 mg of paroxetine.

The liquid composition of the invention can be administered orally by conventional means, e.g., by teaspoons, calibrated vials, etc. Before ingesting the dose of liquid paroxetine composition of the invention, it can be combined with a liquid diluent such as water or other beverage, e.g. fruit juice, coffee, tea, milk, etc., and then ingested. Such a pre-administration dilution would normally be carried out by the patient or medical doctor just before ingesting.

Alternatively, the liquid paroxetine composition could be further diluted by a pharmaceutically acceptable liquid additive such as water or a water/ethanol mixture, to form a medicament; i.e., use the liquid paroxetine composition of the present invention as a concentrated intermediate to make the final oral liquid paroxetine composition. Such a final oral dosage form would typically comprise 10-95% of the concentrate disclosed above and 90-5% of the additive. Such a formation of a second medicament would allow distributors and pharmacists to safely store the paroxetine liquid, in 'concentrate form,' for significant periods and then dispense when needed in a ready to drink form. A single dose of this medicament could be further diluted as part of administration by the patient, such as in a beverage as described above, but would likely be ingested without further dilution; e.g. consuming directly the measured or premeasured dose of the medicament.

The invention will be further described by reference to the following nonlimiting examples.

### Examples

### Example 1.

A liquid composition is made using the following ingredients expressed in amounts per each milliliter of the liquid.

| **Ingredient** | **Amount** |
|---|---|
| Paroxetine-mesylate | 31.31 mg/mL |
| Sodium saccharin | 5 mg/mL |
| Acesulfame-K | 7 mg/mL |
| Mint (14% mint and 86% ethanol) | 22.76 mg/mL |
| Tween 80 (polysorbate) | 10 mg/mL |
| Ethanol (96%) | 143.12 mg/mL |
| Propylene glycol | to volume |

The dry ingredients are weighed into separate vessels. Tween 80 is weighed into a container and thereafter ethanol and mint are weighed into the same container as Tween 80. Stirring is done to dissolve the Tween 80. The pre-weighed dry ingredients are added into the vessel containing the Tween:mint:ethanol mixture and stirring is done. Propylene glycol is added to 75% of the final volume. Stirring is done until the dry ingredients are dissolved and thereafter propylene glycol is added until final volume and stirring is done again.

### Example 2.

A liquid composition is made using the following ingredients expressed in amounts per each mililiter of the liquid.

| **Ingredient** | **Amount** |
|---|---|
| Paroxetine-mesylate | 31.31 mg/mL |
| Sodium saccharin | 5 mg/mL |
| Acesulfame-K | 7 mg/mL |
| Mint (14% mint and 86% ethanol) | 22.76 mg/mL |
| Tween 80 | 10 mg/mL |
| Ethanol (96%) | 143.12 mg/mL |
| Propylene glycol: demi water (2:1) | to volume |

The composition is made by the same procedure as in Example 1, except that a mixture of propylene glycol:H₂O is used instead of pure propylene glycol.

## Claims

1. A liquid paroxetine composition, comprising:
(a) Paroxetine mesylate, in a concentration of at least 5 mg/ml expressed in terms of paroxetine base;
(b) A non-carbohydrate sweetener; and
(c) A solvent which comprises 60-100% of an organic solvent component comprising ethanol, polyol, or mixtures of two or more thereof, and 0-40% of water, wherein the paroxetine composition is in the form of a solution and has a pH less than 7.0,
preferably between 3.5 and 7.0.

2. The liquid composition according to claim 1, wherein said paroxetine concentration is within the range of 10 to 50 mg/ml.

3. The liquid composition according to claim 1-2, wherein said non-carbohydrate sweetener is present in a concentration within the range of 0.1 to 20 mg/ml, preferably 1 to 10 mg/ml.

4. The liquid composition according to claim 1-3, wherein non-carbohydrate sweetener is selected from the group consisting of sodium saccharin, acesulfame potassium, aspartame, sodium cyclamate, thaumatin, sucralose and mixtures thereof.

5. The liquid composition according to claim 1-4, wherein said organic solvent component is selected from the group consisting of ethanol, glycerol, polyethylene glycol, propylene glycol, and combinations of two or more.

6. The liquid composition according to claim 1-5, wherein said organic solvent component contains 0-25% ethanol.

7. The liquid composition according to claim 1-6, wherein said solvent contains no water.

8. The liquid composition according to claim 1-6, wherein said solvent contains 1-20% water.

9. The liquid composition according to claim 1-8, which further comprises a surfactant, a cyclodextrine, or both.

10. The liquid composition according to claim 1-9, for use as a medicament.

11. A process of making a medicament, which comprises combining the liquid composition according to claim 1- 9 with a pharmaceutically acceptable liquid additive.

12. The process according to claim 11, wherein said additive is water or a water/ethanol mixture.

13. The medicament obtainable by the process of claim 11 or 12.

14. An oral liquid paroxetine composition comprising 10-95% of the liquid composition according to claim 1- 9 and 90-5% of a pharmaceutically acceptable liquid additive.

15. The oral liquid paroxetine composition according to claim 14, wherein said additive is water or a water/ethanol mixture.

## Patentansprüche

1. Flüssige Paroxetinzusammensetzung, umfassend:
(a) Paroxetinmesylat in einer Konzentration von mindestens 5 mg/ml, ausgedrückt als Paroxetinbase;
(b) ein Nicht-Kohlenhydratsüßungsmittel; und
(c) ein Lösungsmittel, das 60-100% eines organischen Lösungsmittelbestandteils, der Ethanol, Polyol, oder Gemische von zwei oder mehreren davon umfasst, und 0-40% Wasser umfasst, wobei die Paroxetinzusammensetzung in der Form einer Lösung vorliegt und einen pH von weniger als 7,0,
bevorzugt zwischen 3,5 und 7,0 aufweist.

2. Flüssige Zusammensetzung nach Anspruch 1, wobei die Paroxetinkonzentration innerhalb des Bereichs von 10 bis 50 mg/ml liegt.

3. Flüssige Zusammensetzung nach Anspruch 1-2, wobei das Nicht-Kohlenhydratsüßungsmittel in einer Konzentration innerhalb des Bereichs von 0,1 bis 20 mg/ml, bevorzugt 1 bis 10 mg/ml liegt.

4. Flüssige Zusammensetzung nach Anspruch 1-3, wobei das Nicht-Kohlenhydratsüßungsmittel aus der Gruppe bestehend aus Natriumsaccharin, Acesulfam-Kalium, Aspartam, Natriumcyclamat, Thaumatin, Sucralose und Gemischen davon ausgewählt ist.

5. Flüssige Zusammensetzung nach Anspruch 1-4, wobei das organische Lösungsmittel aus der Gruppe bestehend aus Ethanol, Glycerin, Polyethylenglycol, Propylenglycol und Kombinationen von zwei oder mehreren ausgewählt ist.

6. Flüssige Zusammensetzung nach Anspruch 1-5, wobei der organische Lösungsmittelbestandteil 0-25% Ethanol enthält.

7. Flüssige Zusammensetzung nach Anspruch 1-6, wobei das Lösungsmittel kein Wasser enthält.

8. Flüssige Zusammensetzung nach Anspruch 1-6, wobei das Lösungsmittel 1-20% Wasser enthält.

9. Flüssige Zusammensetzung nach Anspruch 1-8, die weiterhin ein oberflächenaktives Mittel, ein Cyclodextrin oder beides umfasst.

10. Flüssige Zusammensetzung nach Anspruch 1-9 zur Verwendung als ein Medikament.

11. Verfahren zum Herstellen eines Medikaments, das Kombinieren der flüssigen Zusammensetzung gemäß Anspruch 1-9 mit einem pharmazeutisch verträglichen flüssigen Zusatzstoff umfasst.

12. Verfahren nach Anspruch 11, wobei der Zusatzstoff Wasser oder ein Wasser/Ethanolgemisch ist.

13. Medikament, das durch das Verfahren gemäß Anspruch 11 oder 12 erhältlich ist.

14. Orale flüssige Paroxetinzusammensetzung, die 10-95% der flüssigen Zusammensetzung gemäß Anspruch 1-9 und 90-5% eines pharmazeutisch verträglichen flüssigen Zusatzstoffes umfasst.

15. Orale flüssige Paroxetinzusammensetzung nach Anspruch 14, wobei der Zusatzstoff Wasser oder ein Wasser/Ethanolgemisch ist.

## Revendications

1. Une composition liquide de paroxétine, comprenant:
(a) du mésylate de paroxétine, en une concentration d'au moins 5 mg/ml exprimée sous la forme de paroxétine base;
(b) un édulcorant non glucidique ; et
(c) un solvant qui comprend 60-100% d'un solvant organique comprenant de l'éthanol, du polyol ou des mélanges de deux ou plusieurs d'entre eux, et 0-40% d'eau,
laquelle composition de paroxétine est sous forme d'une solution et présente un pH inférieur à 7,0, de préférence compris entre 3,5 et 7.0.

2. La composition liquide selon la revendication 1, dans laquelle ladite concentration en paroxétine est comprise entre 10 et 50 mg/ml.

3. La composition liquide selon la revendication 1-2, dans laquelle ledit édulcorant non glucidique est présent en une concentration comprise entre 0,1 et 20 mg/ml, de préférence entre 1 et 10 mg/ml.

4. La composition liquide selon la revendication 1-3, dans laquelle ledit édulcorant non glucidique est choisi dans le groupe formé par la saccharine sodique, l'acésulfame de potassium, l'aspartame, le cyclamate de sodium, la thaumatine, le sucralose et les mélanges entre eux.

5. La composition liquide selon la revendication 1-4, dans laquelle ledit solvant organique est choisi dans le groupe formé par l'éthanol, le glycérol, le polyéthylene glycol, le propylène glycol, et les mélanges entre deux ou plusieurs d'entre eux.

6. La composition liquide selon la revendication 1-5, dans laquelle ledit solvant organique contient 0-25% d'éthanol.

7. La composition liquide selon la revendication 1-6, dans laquelle ledit solvant ne contient pas d'eau.

8. La composition liquide selon la revendication 1-6, dans laquelle ledit solvant contient 1 à 20% d'eau

9. La composition liquide selon la revendication 1-8, qui comprend en outre un agent tensioactif, une cyclodextrine ou l'un et l'autre.

10. La composition liquide selon la revendication 1-9, pour une utilisation en tant que médicament.

11. Un procédé de fabrication d'un médicament, qui comprend la combinaison de la composition liquide selon la revendication 1- 9 avec un additif liquide pharmaceutiquement acceptable.

12. Le procédé selon la revendication 11, dans laquelle ledit additif est de l'eau ou un mélange eau/éthanol.

13. Le médicament susceptible d'être obtenu par le procédé de la revendication 11 ou 12.

14. Une composition de paroxétine liquide administrable par voie orale comprenant 10-95% de la composition liquide selon les revendications 1- 9 et 90-5% de l'additif liquide pharmaceutiquement acceptable.

15. Une composition de paroxétine liquide administrable par voie orale selon la revendication 14, dans laquelle ledit additif est de l'eau ou un mélange eau/éthanol.
